Europäisches Patentamt

European Patent Office

Office européen des brevets

(11) Publication number: **0 352 330**
A1

## (12) EUROPEAN PATENT APPLICATION
### published in accordance with Art. 158(3) EPC

(21) Application number: 88909382.9

(22) Date of filing: 28.10.88

(86) International application number:
PCT/JP88/01099

(87) International publication number:
WO 89/04339 (18.05.89 89/11)

(51) Int. Cl.⁴: **C08H 1/00 , C08L 89/00 ,
A61L 33/00 , A61K 31/725**

(30) Priority: 02.11.87 JP 277842/87
02.11.87 JP 277843/87

(43) Date of publication of application:
**31.01.90 Bulletin 90/05**

(84) Designated Contracting States:
**BE DE FR GB IT NL SE**

(71) Applicant: **Terumo Kabushiki Kaisha
No. 44-1, Hatagaya 2-chome Shibuya-ku
Tokyo 151(JP)**

(72) Inventor: **HIRABAYASHI, Kiyoshi
16-21, Ogawahigashimachi 1-chome
Kodaira-shi Tokyo 187(JP)**

(74) Representative: **Gillard, Marie-Louise et al
Cabinet Beau de Loménie 55, Rue
d'Amsterdam
F-75008 Paris(FR)**

(54) FIBROIN MOLDINGS, PROCESS FOR THEIR PREPARATION, HEPARIN-IMMOBILIZING CARRIER, AND PROCESS FOR ITS PREPARATION.

(57) A fibroin molding is obtained by treating fibroin with a crosslinking or coupling agent such as carbodiimide or glutaraldehyde. This molding can be formed into any desired form such as cast film, regenerated fibers, permeable membrane, coating membrane, porous material, microbeads, etc. They can be prepared by adding the coupling agent to a fibroin solution and solidifying fibroin, or by solidifying and molding fibroin from a fibroin solution and treating the product with the coupling agent. The above-described molding can be used by immobilizing thereon heparin or the like.

EP 0 352 330 A1

1

SPECIFICATION

TITLE OF THE INVENTION

Molded Fibroin Product, Method for Producing Same, Immobilized-Heparin Carrier and the Method for Producing Same

BACKGROUND OF ART

This invention relates to a molded fibroin product having excellent expandability, flexibility and biocompatibility, and the method for producing the same.

The present invention also relates to an immobilized heparin carrier exhibiting prolonged duration of anti-thrombotic properties and excellent biocompatibility, and the method for producing the same.

Silk yarn has been used from old for apparels as natural fibers exhibiting soft touch, feeling and high gloss. Fibroin, its main constituent, has come to be used in recent years as tne moldable natural protein as the starting material for cosmetics or medicaments. For example, fibroin is used singly or in combination with other compounds, as the enzyme containing membrane, as disclosed in the Japanese Patent Application KOKAI No. 55-81589; as the fixed enzyme carrier, as disclosed in the Japanese Patent Application KOKAI No. 56-15687; as the poprous film, as disclosed in the Japanese Patent Application KOKAI No. 56-40156; as solid type perfume, as disclosed in the Japanese patent Application KOKAI No. 56-163658; as hydrophilic porous body, as disclosed in the Japanese Patent Application KOKAI No. 56-166235; as packing agent, as disclosed in the Japanese Patent Application KOKAI No. 57-77612; as liquid medicament or lotion, as disclosed in the Japanese Patent Application KOKAI No. 57-175110; as water absorbant, as disclosed in the Japanese Patent Application KOKAI No. 58-244; as cataplasm agent, as disclosed in the Japanese Patent Application KOKAI No. 59-27821; as foamed body, as disclosed in the Japanese Patent Application KOKAI No. 59-21339; as an immobilized antibody carrier, as disclosed in the Japanese Patent Application KOKAI No. 60-142259; as immobilized antigen/antibody carrier, as disclosed in the Japanese Patent Application KOKAI No. 60-155129; as a medicament carrier, as disclosed in the Japanese Patent Application No. 60-155125; or as makeup cosmetics, as disclosed in the Japanese Patent Application KOKAI No. 62-415.

When used for the above application, fibroin is produced from an aqueous solution of fibroin as the starting material. This aqueous solution of fibroin is produced by removing sericin from cocoon, silk yarn, cocoon debris or silk yarn debris, dissolving the remaining mass in an aqueous solution containing alkali metals or alkali earth metals and desalting the resulting solution such as by dialysis.

This aqoeous solution of fibroin is molded or formed by itself or in combination with other compounds to a desired form and desired properties to produce the desired product. At any rate, it is necessary for the water-soluble fibroin to be converted into a water insoluble fibroin.

One of the methods used for this purpose is by elevating the degree of cristalinity. More specifically, there are known the random coil state, silk I type crystals or $\alpha$ type structure and a silk II type crystal or $\beta$ type structure, as the molecular form of fibroin, as stated in O. Kratkey et al., Nature, 165, 475 (1950). Since these molecular forms are insoluble in water with the exception of the Silk II type crystals, it is necessary to raise the crystallization fraction of the Silk II type crystals by some means or other to render these crystals insoluble in water.

As means for this purpose, there are known methods by addition of diols or polyalcohols, as disclosed in the Japanese Patent Application KOKAI No. 55-81589; by low temperature freeze drying, as disclosed in the Japanese Patent Application KOKAI No. 56-40156; or by using the pH of 3.0 to 5.0, addition of lower alcohols, or agitation at a higher shear deformation speed or in ultrasonic processing, as disclosed in the Japanese Patent Application KOKAI No. 56-163658.

There have also been devised methods by grafting synthetic polymers to the crystals according to usage and application, instead of by raising the degree of crystallinity, as disclosed in the Japanese Patent Application KOKAI No. 57-77612; combining the crystals with the synthetic polymer, as disclosed in the Japanese Patent Application KOKAI No. 58-244; or by combining the rubber of the main component with fibroin, as disclosed in the Japanese Patent Application No. 59-21339.

However, these prior-art methods are unsatisfactory in the following respects.

That is, fibroin with increased degree of crystallinity has a higher strength but lacks in elongation and ductility. Also the biocompatibility of fibroin, which is desired to be produced, is reduced by one half due to the synthetic polymer with which it is combined. When fibroin is crystallized by, for example, agitation, most of fibroin is precipitated to lower the yield. The pH value set to 3.0 to 5.0 or ultrasonic processing results in modification or denaturation of fibroin.

On the other hand, the molded fibroin product produced as above has a higher fraction of crystallization with respect to the β type crystals, so that it exhibits a denser molecular packing than that of the product exhibiting a lower value of the fraction of crystallization with respect to the β type crystals. Thus the product is poor in

4

properties thereof such as water absorption or material transmitting properties.

Also the above described prior-art methods reside simply in that biocompatibility is naturally expected from fibroin which is the material derived from organisms, or in that enzymes or medicaments are mixed into the starting fibroin material to exhibit the functions proper to fibroin.

Thus these methods cannot be said to provide for biocompatibility or functional properties positively.

Also, various materials to which is immobilized heparin as the anti-thrombotic material have been devised. For example, there have been devised a method of heparinating the high molecular weight surface by ionic bond using a surfactant such as tridodecylmethyl ammonium chloride, as disclosed in "Heparin", Kodan-sha Scientific, p.p. 219 to 223, or a method of immobilizing heparin by covalent bond to the high molecular weight surface.

However, with ionic bond, heparin is eluated quickly into the blood, such that it is ultimately lost in a shorter time. In both the covalent bond and ionic bond, the carrier polymer is disadvantageously specified from the viewpoint of biocompatibility.

It is therefore a principal object of the present invention to overcome the above described drawbacks of the prior art methods and to provide a molded fibroin product which is superior in flexibility and expandability or stretchability and which is also superior in biocompatibility.

It is another object of the present invention to overcome the above described drawbacks of the prior art methods and to provide an immobilized heparin carrier having prolonged duration in anti-thrombotic properties and excellent biocompatibility, and the method for producing the immobilized heparin carrier.

DISCLOSURE OF THE INVENTION

Conventionally, insolubility in water was attained by improving the fraction of crystallization of β type crystals. However, because of the dense molecular packing, fibroin when used as the membrane presented only low transmitting properties to water or solute. Also, when fibroin is used as the carrier for medicaments, there was not much space left through which the medicament can be intruded into the carier.

It has now been found that the random coil state or α type crystal structure can be rendered insoluble in water through cross-linking. This finding has led to completion of the present invention.

According to a first aspect of the present invention, there is provided a molded fibroin product essentially comprising fibroin wherein fibroin is processed with a cross-linking agent.

According to a second aspect of the present invention, there is provided a method for producing a molded fibroin product comprising addition of a cross-linking agent to a fibroin solution followed by solidification of fibroin.

According to a third aspect of the present invention, there is provided a method for producing a molded fibroin product comprising solidifying and molding fibroin from a fibroin solution followed by treatment with a cross-linking agent.

In the present invention, stated above, the cross-linking agent is preferably carbodiimide or glutar aldehyde.

The molded products may be in a variety of morphologic forms, such as, for example, cast films, regenerated fibers, osmotic membranes, coated films, porous bodies or microbeads.

According to a fourth aspect of the present invention, there is provided a heparin immobilized carrier characterized in that heparin is fixed to fibroin by a coupling agent.

According to a fifth aspect of the present invention, there is provided a method for producing a heparin immobilized carrier comprising insolubilizing fibroin from the fibroin solution and processing the insolubilized fibroin with a coupling agent and heparin to fix heparin to the fibroin with the coupling agent.

According to a sixth aspect of the prtesent invention, there is provided a method for producing a heparin immobilized carier comprising adding a coupling agent and heparin to a fibroin solution to fix heparin to fibroin with the intermediary of the coupling agent and insolubilizing fibroin.

In the present invention, as stated above, the coupling agent is preferably carbodiimide or glutar aldehyde.

BEST MODE OF PRACTICING THE INVENTION

The molded fibroin product and the method for producing the molded fibroin product according to the present invention will be explained hereinbelow in more detail.

First of all, the method for producing the molded fibroin product according to the present invention is explained. According to the present invention, an aqueous solution of fibroin is first produced. As the starting materials for the aqueous solution of fibroin, fibroin sources such as cocoon, silk yarn, cocoon debris, silk yarn debris, spun silk yarn or silk waste, are employed. These starting materials, freed of cerisine by purification in a known manner, are dissolved in, for example, a copper-ammonia aqueous solution, copper hydroxide-ethylene diamine aqueous solution, aqueous rhodanate solution, lithium bromide aqueous solution, calcium chloride solution, aqueous calcium nitrate solution or a magnesium nitrate solution, dialyzed with respect to, for example, water, and desalted to produce an aqueous fibroin solution. This solution may be adjusted to a desired concentration.

During the dissolution process, alcohols may be added to the system to improve solubility.

According to the present invention, a suitable amount of a cross-linking agent is added to the thus prepared aqueous fibroin solution for cross-linking the fibroin. The reaction temperature is preferably the ambient temperatue to avoid denaturation or modification. In this manner, water-soluble fibroin is rendered insoluble in water. The cross-linking agents that may be applied to the aqueous fibroin solution preferably include 1-ethyl-3-(3-dimethylaminopropyl) carboimide chlorate (water soluble carbodiimide or EDC), glutar aldehyde (GA), epoxy compounds, bisimidates, diazides, dinitrenes, dihalides, dialdehydes, dimaleimides, diisocyanates, diepoxides, disulphonyl chlorides, carbodiimides, isooxazolium salts, di(1, 10-phenanthroline) copper, dithiobis (succin-imidyl propionic acid) or a Woodward reagent. Meanwhile, EDC, marketed in the form of chlorates, is thought to be dissociated in water and reacted with fibroin in the form of carbodiimide.

After fibroin is cross-linked in the above described manner, it is molded to the desired shape.

According to an alternative method of the present invention, the aqueous fibroin solution produced as above is molded to a desired form without adding cross-linking agents thereto, and the water-soluble fibroin which as yet is at the low stage of crystallization is then reacted with the cross-linking agent. This allows water-soluble fibroin to be insloubilized in water.

The cross-linking agents employed at this time include diisocyanates and diepoxy compounds.

The cross-linked molded product obtained in the above manner may then be processed with lower alcohols or heat-treated, if so desired. According to the usages of the molded product, fibroin may be chemically modified or admixed with additives in advance or, alternatively, the molded fibroin product may be chemically modified or admixed with additives.

The molded product obtained from the aqueous fibroin solution may be dried by heat treatment or using a dehydrating agent, such as alcohols.

After being cross-linked by various methods, such as those described above, fibroin may be molded to various morphologic forms, depending on the usages and applications.

As shown in the Examples to follow, the cross-linked molded fibroin product of the present invention exhibits superior flexibility and stretchability. Also, silk yarn is inherently superior in biocompatibility, as may be demonstrated by its application to suturing yarn. The molded fibroin product of the present invention is similarly superior in biocompatibility.

The molded product may be processed to various morphologic forms, according to its usages and applications.

For example, the molded product may be formed into films. For forming films from the above described aqueous fibroin solution, the casting method or other methods such as the regenerating and solidifying methods may be employed.

The molded fibroin product in the form of films may be used as the filter or sensor membranes. The molded product may also be in the form of fibers. Fibers may be produced by any known methods from the aqueous fibroin solution and may be used as the suturing yarn, artificial blood vessel or non-woven fabrics.

The molded product may also be in the form of membranes which may be used for osmosis and as films applied to other substrates. Examples of the usage of the membranes include dialytic membranes and improving the bioadaptabilty of the substrates. The films on other substrates may be formed such as by coating and drying.

The molded product may also be formed into agglomerates, such as microbeads or pellets. It may also be formed into a porous body such as by addition of a foaming agent or by freeze drying or formed into microbeads such as by drying and crushing. Slow release poperties may be improved by having the medicament or the like carried by the molded product. The usage of the molded product also includes that as the base material for adsorbents.

It is thought that the molded fibroin product of the present invention is processed with the cross-linking agent and thereby insolubilized so that it is not of the β type structure. The random coil state and the α type structure are dissolved in the molded state since they are soluble in water. With the cross-linked molded fibroin product of the present invention, the random coil state and the α type structure are simply cross-linked without being of the β type structure, so that they are swollen but are not dissolved when immersed in water.

The immobilized-heparin carrier of the present invention and the method for producing the same will be explained hereinbelow in more detail.

First of all, the method for producing the molded fibroin product according to the present invention is explained. According to the present invention, an aqueous solution of fibroin is first produced. As the starting materials for the aqueous solution of fibroin, fibroin sources such as cocoon, silk yarn, cocoon debris, silk yarn debris, spun silk yarn or silk waste, are employed. These starting materials, freed of cerisine by purification in a known manner, are dissolved in, for example, a copper-ammonia aqueous solution, copper hydroxide-ethylene diamine aqueous solution, aqueous rhodanate solution, lithium bromide aqueous solution, calcium chloride solition, aqueous calcium nitrate solution or a magnesium nitrate solution, dialyzed with respect to, for example, water, and desalted to produce an aqueous fibroin solution. This solution may be adjusted to a desired concentration.

During dissolution process, alcohols may be added to the system to improve solubility.

According to the present invention, a suitable amount of a coupling agent and heparin are added to the aqueous solution of fibroin thus produced to fix heparin to fibroin by the medium of the coupling agent. The reaction temperature at this time is preferably the ambient temperature to avoid denaturation. The coupling agents applied to the aqueous solution of fibroin include 1-ethyl-3-(3-dimethylaminopropyl) carbodiimide chlorate (water-soluble carbodiimide: EDC), glutar aldehyde (GA), epoxy compounds and isooxazolium salts. Meanwhile, EDC, marketed in the form of chlorates, is thought to be dissociated in water and reacted with fibroin in the form of carbodiimide.

After fibroin is coupled in the above manner, it is molded to the desired shape.

According to an alternate method of the present invention, water-soluble fibroin is rendered insoluble in water without adding the coupling agent and heparin to the aqueous fibroin solution produced as described above. Subsequently, the coupling agent and heparin are added to and reacted with fibroin thus insolubilized in water to fix heparin to fibroin by the medium of the coupling agent. The methods for insolubilizing fibroin may be by addition of lower alcohols, such as an aqueous solution of methanol or ethanol, stretching or by heat treatment.

When the coupling agent and heparin are added to the aqueous fibroin solution to fix heparin to fibroin, part of heparin is taken into the inside of the fibroin crystal.

However, when the coupling agent and heparin are added after fibroin is once insolubilized in water, heparin is fixed to the fibroin surface to realize effective utilzation of heparin. Therefore it is more preferred to cause the coupling agent and heparin to act after fibroin is once insolubilized in water.

The immobilized-heparin carrier obtained in the above manner may then be processed with lower alcohols or heat-treated, as desired. According to the usages of the molded product, fibroin may be chemically modified or admixed with additives in advance. Alternatively, it may be the molded product that is previously chemically modified or admixed with additives.

The heparin-immobilized carrier obtained from the aqueous fibroin solution may be dried by heat treatment or by air drying.

After heparin is fixed to fibroin by the above described method or other various methods, fibroin may be molded to various morphologic forms, depending on the usages and applications.

As shown in the Examples to follow, the cross-linked molded fibroin product of the present invention exhibits prolonged duration of the anti-thrombotic operation. Also, silk yarn is inherently superior in biocompatibility, as may be demonstrated by its application to suturing yarn. The heparin-immobilized molded fibroin product of the present invention is similarly superior in bioaffinity.

The heparin-immobilized molded product may be processed to various morphologic forms, according to its usages and applications.

For example, the molded product may be formed into films. For forming films from the above described aqueous fibroin solution, the casting method or other methods such as the extrusion molding methods may be employed. The film-shaped molded fibroin product may be used as the blood container or the like.

The molded product may also be in the form of fibers. Fibers may be produced by any known methods from the aqueous fibroin solution and may be used as the filter or as suturing yarn.

The molded product may also be in the form of membranes which may be used for osmosis and as films applied to other substrates. Examples of the usage of the membranes include blood processing membranes. The membranes may be formed on other substrates such as by coating and drying.

The molded product may also be formed into agglomerates, such as microbeads or pellets. It may also be formed into a porous body such as by addition of a foaming agent or by freeze drying or formed into microbeads such as by drying and crushing. Slow release poperties may be improved by having the medicament or the like carried by the molded product. The usage of the molded product also includes that as the base material for adsorbents. It is thought that the immobilized heparin molded fibroin product of the present invention is insolubilized in water but has lesser contents of the β type crystals. The random coil state and the α type structure are dissolved in the molded state since they are soluble in water. The cross-linked molded fibroin product of the present invention is swollen but is not dissolved when immersed in water. Since heparin fixed to fibroin is released or dissociated gradually, the function of heparin (anti-thrombotic properties) of heparin may be maintained for prolonged time.

The present invention will be explained in more detail with reference to the Examples and Comparative Examples.
(Example 1)

Silk yarn was introduced into a 50-times of volume of a 0.5%-aqueous solution of sodium carbonate and stirred slowly at 85 to 90°C for 20 minutes. The resulting mass was washed twice with the same volume of water and then washed with flowing water to remove cerisine. This silk yarn was further subjected to Soxlet extraction with a liquid benzene/methanol = 2/1 to remove wax and the coloring matter.

3 g of the thus refined silk yarn was immersed in 100 ml of 8M LiBr aqueous solution and allowerd to stand at 37°C for 48 hours stationarily to effect dissolution. This solution was introduced into a cellulose tube for dialysis to effect dialysis with respect to water. The dialytic liquid was exchanged thrice a day and silver nitrate was dissolved in the dialytic solution to perform the operation until white turbidity was no longer observed. The dialytic tube was taken out in its entirety and dried in air by directing an air flow thereto to concentrate the aqueous fibroin solution. The solution was centrifuged for 10 minutes at about 9000G to remove the solid matter. The concentration of this aqueous fibroin solution was 1.5 wt%.

The solution was then divided into two parts, to one of which a peptide synthetic reagent, namely 1-ethyl-3-(3-dimethylaminopropyl) carbodiimde chlorate, referred to hereinafter as EDC, was added as the cross-linking agent in an amount equal to that of fibroin to effect dissolution. The solution admixed with EDC was used in the inventive Example 1 and the solution not admixed with EDC was used in the Comparative Example 1.

Both of these solutions were caused to flow into a polystyrene Schale and dried at room temperature for three days. A fibroin film was formed in each Schale and could be peeled off from the Schale for both solutions. These films are partially cut off and immersed in water. The solution admixed with EDC was swollen but was not dissolved. The solution not admixed with EDC was dissiolved readily in water.

Both of the solutions were immersed in ethanol overnight and dried. The produced films were again partially cut off and injected into water. Neither of these films was now dissolved in water.

Both of the films were subjected to a tensile test. The sample was cut to a size of 80 mm length, 10 mm width and 0.04 mm thickness and the fracture strength and elongation were measured at a temperature of 23°C, initial length of 50 mm and a tensile rate of 50 mm/min. using a strograph T manufactured by the Toyo Seiki Co. Ltd. The results are shown below.

|  | fracture strength (g/cm2) | fracture elongation (%) |
|---|---|---|
| Ex. 1 | $4.3 \times 10^4$ | 60 |
| Comp. Ex. 1 | $11.5 \times 10^4$ | 2 |

Thus it is seen that, in the inventive Example 1, the elongation reaches 30 times that of the Comparative Example 1, although the strength is lowered to 37% of that of the Comparative Example 1, and that, while the product of the Comparative Example 1 is tough and brittle, the product of the inventive Example 1 is soft and excellent in stretchability.

(Example 2)

Silk yarn was introduced into a 50-times of volume of a 0.5%-aqueous solution of sodium carbonate and stirred slowly at 85 to 90°C for 20 minutes. The resulting mass was washed twice with the same volume of water and then washed with flowing water to remove cerisine. This silk yarn was further subjected to Soxlet extraction with a liquid benzene/methanol = 2/1 to remove wax and the coloring matter.

3 g of the thus refined silk yarn was immersed in 100 ml of 8M LiBr aqueous solution and allowerd to stand at 37°C for 48 hours stationarily to effect dissolution. This solution was introduced into a cellulose tube for dialysis to effect dialysis with respect to water. The dialytic liquid was exchanged thrice a day and silver nitrate was dissolved in the dialytic solution to perform the operation until white turbidity was no longer observed. The dialytic tube was taken out in its entirety and dried in air by directing an air flow thereto to concentrate the aqueous fibroin solution. The solution was centrifuged for 10 minutes at about 9000G to remove the solid matter.

This fibroin solution was cast in a polystyrene Schale and dried at room temperature to produce a fibroin film. This film as it is soluble in water, so that it is treated with ethanol and thereby rendered insoluble in water for use in the experiment.

The fibroin film was dipped in the inventive Example 2 in a solution in 50 ml of a citric acid-phosphate buffer, pH 3.2, of each 500 mg of heparin and 1-ethyl-3-(3-dimethylamonipropyl) carbodiimide chlorate, referred to hereinafter as EDC, and, in Comparative Example 2, in a solution in which only 500 mg of heparin only was similarly dissolved. Incubation was then performed at 25°C for five hours. The films were then taken out and dipped in 250 ml of distilled water to measure the changes in the eluated amount of heparin with lapse of time.

(Example 3)

A fibroin film was prepared similarly to Example 2 and dipped in citric acid-phospahte buffer, pH 3.2, containing 200 mg of heparin dissolved therein, and incubation was carried out at 37°C for 24 hours. One half of the film was taken out by way of Comparative Example 3 and heparin was eluated in a citric acid-phosphate buffer, pH 7.4. By way of the inventive Example 3, glutar aldehyde, referred to hereinafter as GA, was added to the remaining half of the film so as to give a concentration of 0.025 M, and incubation was performed for 24 hours. The films were then transferred into a citric acid-phosphate buffer, pH 7.4, containing GA at a concentration of 0.025 M, and maintained thereat for 24 hours. After washing with water, the films

16

were shifted into a 2 wt% dimedone solution and excess unreacted GA was removed (24 hours). After washing with water, heparin was eluated in a citric acid-phosphate buffer, pH 7.4, and changes in the amount of eluated heparin were measured.

| <results> | eluated heparin [IU] |
|---|---|
| Ex.2 | 1300 |
| Comp. Ex.2 | 2200 |
| Ex.3 | 3 |
| Comp. Ex.3 | 300 |

In both of the inventive Examples 2 and 3, the eluated amount of heparin is controlled as compared to the Comparative Examples 2 and 3, and heparin is immobiliaed to fibroin.

(Example 4)

Changes with lapse of time of the amount of eluated heparin into water from the fibroin film obtained in the inventive Example 2 and the Comparative Example 2 were measured. It is noted that the absolute value of the amount of eluated heparin is small since the experiment was conducted on a reduced scale as compared to Example 1. The results are shown in the following Table.

| | <Eluated Heparin [IU]> | |
|---|---|---|
| time of eluation [hr] | Ex. 2 | Comp. Ex. 2 |
| 3 | 45 | 68 |
| 12 | 59 | 82 |
| 24 | 67 | 88 |
| 72 | 81 | 88 |

In the Comparative Example 2, the amount of eluation reaches saturation in 12 hours, whereas, in the inventive Example 2, the amount of eluation reaches saturation in 72 hours, indicating the prolonged duration of heparin.

INDUSTRIAL UTILIZABILITY

The molded fibroin product of the present invention shows stretchability 30 times that of the conventional molded fibroin product that lacks in stretchability and flexibility. Such molded fibroin product may be obtained easily by the method of the present invention.

In the heparin-immobilized carrier of the present invention, heparin is immobilized to fibroin using carbodiimide or glutar aldehyde. When heparin is simply contained in fibroin, heparin is eluated easily and the effect cannot be maintained, whreras the immobilized carrier of the present invention, not only contains heparin but is also bonded to heparin, so that the effect is maintained and the carrier is useful as the anti-thrombotic material.

Such heparin immobilized carrier may be produced easily in accordance with the method of the present invention.

WHAT IS CLAIMED IS:

(1) A molded fibroin product essentially comprising fibroin wherein fibroin is processed with a cross-linking agent.

(2) The molded fibroin product according to claim 1 wherein said cross-linking agent is carbodiimide.

(3) The molded fibroin product according to claim 1 wherein said cross-linking agent is glutar aldehyde.

(4) The molded fibroin product according to any one of claims 1 to 3 wherein said molded fibroin product is a cast film.

(5) The molded fibroin product according to any one of claims 1 to 3 wherein said molded fibroin product is regenerated fibers.

(6) The molded fibroin product according to any one of claims 1 to 3 wherein said molded fibroin product is an osmotic membrane.

(7) The molded fibroin product according to any one of claims 1 to 3 wherein said molded fibroin product is a coated film.

(8) The molded fibroin product according to any one of claims 1 to 3 wherein said molded fibroin product is a porous body.

(9) The molded fibroin product according to any one of claims 1 to 3 wherein said molded fibroin product is microbeads.

(10) A method for producing a molded fibroin product comprising addition of a cross-linking agent to a fibroin solution followed by solidification of fibroin.

(11) A method for producing a molded fibroin product comprising solidifying and molding fibroin from a fibroin solution followed by treatment with a cross-linking agent.

(12) A heparin-immobilized carrier characterized in that heparin is fixed to fibroin by a coupling agent.

(13) The heparin-immobilized carrier according to claim 12 wherein said coupling agent is carbodiimide.

(14) The heparin immobilized carrier according to claim 12 wherein said coupling agent is glutar aldehyde.

(15) A method for producing a heparin-immobilized carrier comprising insolubilizing fibroin from the fibroin solution and processing the insolubilized fibroin with a coupling agent and heparin to fix heparin to the fibroin with the intermediary of the coupling agent.

(16) A method for producing a heparin immobilized carier comprising adding a coupling agent and heparin to a fibroin solution to fix heparin to fibroin with the intermediary of the coupling agent, and insolubilizing fibroin.

# INTERNATIONAL SEARCH REPORT

International Application No PCT/JP88/01099

**I. CLASSIFICATION OF SUBJECT MATTER** (if several classification symbols apply, indicate all) [6]

According to International Patent Classification (IPC) or to both National Classification and IPC

Int.Cl[4]  C08H1/00, C08L89/00, A61L33/00, A61K31/725

**II. FIELDS SEARCHED**

| Minimum Documentation Searched [7] | |
|---|---|
| Classification System | Classification Symbols |
| IPC | C08H1/00-1/06, C08L89/00-89/06, A61L33/00, A61K31/725 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, B2, 53-24227 (Idemitsu Kosan Co., Ltd.) 19 July 1978 (19. 07. 78) Column of Claim (Family: none) | 1-11 |
| Y | JP, A, 61-502129 (University of Medicine and Dentistry of New Jersey) 25 September 1986 (25. 09. 86) Column of Claim & WO, A1, 8504413 & IL, A0, 74715 | 12-16 |
| Y | Tanaka Yoshio [Yuki Seizo Kogyo Kagaku (last volume)] 26 June 1935 (26. 06. 35) Maruzen (Tokyo)  P.502-504 | 1-16 |

* Special categories of cited documents: [10]

"A" document defining the general state of the art which is not considered to be of particular relevance

"E" earlier document but published on or after the international filing date

"L" document which may throw doubts on priority claim(s) or which is cited- to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 17, 1989 (17. 01. 89) | January 30, 1989 (30. 01. 89) |
| International Searching Authority | Signature of Authorized Officer |
| Japanese Patent Office | |

Form PCT/ISA/210 (second sheet) (January 1985)